(19) Europäisches Patentamt
European Patent Office
Office européen des brevets

(11) EP 4 431 939 A1

(12) EUROPEAN PATENT APPLICATION
published in accordance with Art. 153(4) EPC

(43) Date of publication:
**18.09.2024 Bulletin 2024/38**

(21) Application number: **22888879.8**

(22) Date of filing: **01.07.2022**

(51) International Patent Classification (IPC):
***G01N 33/00*** (2006.01) ***C01B 15/023*** (2006.01)

(52) Cooperative Patent Classification (CPC):
**C01B 15/023;** B01J 10/002

(86) International application number:
**PCT/CN2022/103300**

(87) International publication number:
**WO 2023/077844 (11.05.2023 Gazette 2023/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2021 CN 202111313752**

(71) Applicant: **Nanjing Institute of Microinterface Technology Co., Ltd**
**Nanjing, Jiangsu 210047 (CN)**

(72) Inventors:
- **ZHANG, Zhibing**
  **Nanjing, Jiangsu 210047 (CN)**
- **ZHOU, Zheng**
  **Nanjing, Jiangsu 210047 (CN)**
- **ZHANG, Feng**
  **Nanjing, Jiangsu 210047 (CN)**
- **LI, Lei**
  **Nanjing, Jiangsu 210047 (CN)**
- **TIAN, Hongzhou**
  **Nanjing, Jiangsu 210047 (CN)**
- **WEI, Gelin**
  **Nanjin, Jiangsu 210047 (CN)**
- **LU, Wenjun**
  **Nanjing, Jiangsu 210047 (CN)**
- **LI, Xiabing**
  **Nanjing, Jiangsu 210047 (CN)**
- **LI, Yongqiang**
  **Nanjing, Jiangsu 210047 (CN)**

(74) Representative: **Zaboliene, Reda**
**Metida**
**Business center Vertas**
**Gyneju str. 16**
**01109 Vilnius (LT)**

# (54) METHOD FOR EVALUATING MICROBUBBLE ENHANCEMENT IN SYSTEM FOR PREPARING HYDROGEN PEROXIDE BY MEANS OF ANTHRAQUINONE PROCESS

(57) The invention provides an evaluation method for micro-bubble strengthening in a hydrogen peroxide system prepared by anthraquinone method, including the following steps: (a) establishing an oxidation reaction equation; (b) constructing a macroscopic reaction kinetic equation of reaction gas; (c) constructing a mathematical expression of an oxygen reaction enhancement factor $E_B$; (d) constructing the calculation method of the concentration of each reaction components in the liquid phase, and calculating the concentration of each reaction component in the liquid phase. The present invention discusses the influence of bubble size on gas-liquid phase interface area, volume mass transfer coefficient and macroscopic reaction rate by constructing a structure-effect controlled mathematical model of a hydrogen peroxide oxidation reaction system prepared by anthraquinone method and evaluates the strengthening effect of micro-interface oxidation reaction. It provides a basis for the design and operation of micro-interface reaction strengthening reactors. The reactor designed by the evaluation method of the present invention can solve the problem of uneven gas-liquid distribution and enhance mass transfer at the same time, thereby maximizing the space-time yield and realizing the micro-interface economics of reaction strengthening technology.

EP 4 431 939 A1

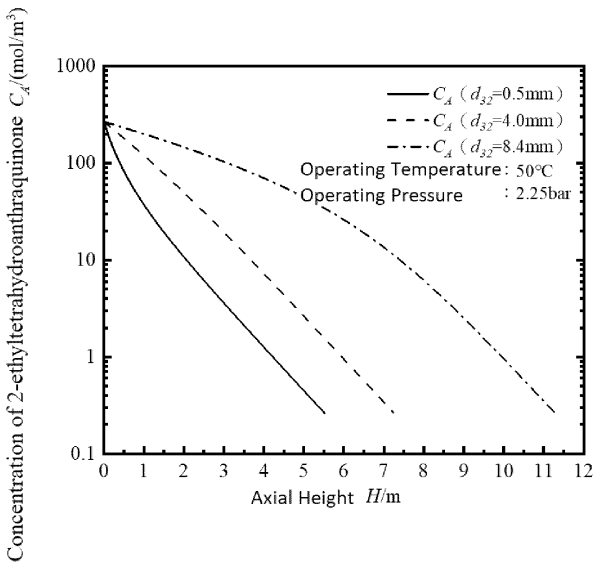
Concentration of 2-ethyltetrahydroanthraquinone $C_A$/(mol/m$^3$)
$C_A$ ($d_{32}$=0.5mm)
$C_A$ ($d_{32}$=4.0mm)
$C_A$ ($d_{32}$=8.4mm)
Operating Temperature: 50°C
Operating Pressure : 2.25bar
1000
100
10
1
0.1
0 1 2 3 4 5 6 7 8 9 10 11 12
Axial Height $H$/m

Fig. 3

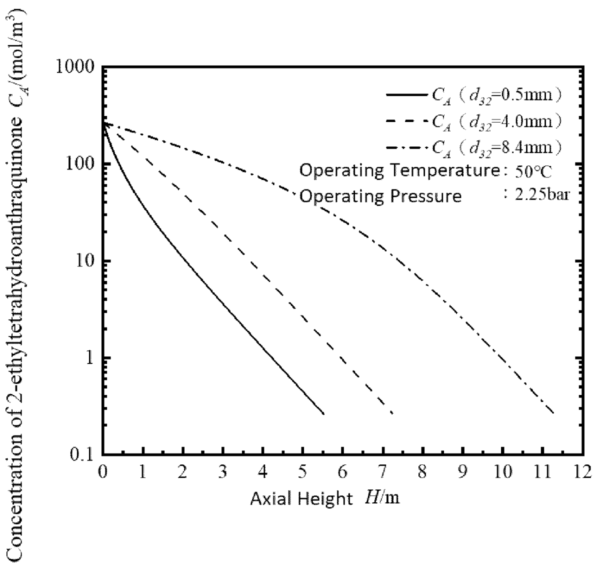
Concentration of 2-ethyltetrahydroanthraquinone $C_A$/(mol/m$^3$)
$C_A$ ($d_{32}$=0.5mm)
$C_A$ ($d_{32}$=4.0mm)
$C_A$ ($d_{32}$=8.4mm)
Operating Temperature: 50°C
Operating Pressure : 2.25bar
1000
100
10
1
0.1
0 1 2 3 4 5 6 7 8 9 10 11 12
Axial Height $H$/m

Fig. 3

## Description

## FIELD OF THE INVENTION

The present invention relates to the technical field of micro-interface strengthening, in particular to an evaluation method for micro-bubble strengthening in a hydrogen peroxide system prepared by anthraquinone method.

## BACKGROUND OF THE INVENTION

Using anthraquinone to produce hydrogen peroxide has become a mature and reliable hydrogen peroxide industrial production technology, and it is the most widely used mainstream production process in large-scale industrial production. The basic process flow is: the working liquid is catalyzed by the palladium catalyst and hydrogen is added to generate a hydrogenated liquid containing hydrogen anthraquinone; the hydrogenated liquid reacts with oxygen to generate an oxidized liquid containing anthraquinone and hydrogen peroxide; the oxidized liquid is extracted with pure water to obtain a raffinate and a crude dilute hydrogen peroxide; the crude dilute hydrogen peroxide is purified with aromatics to obtain dilute hydrogen peroxide; the raffinate is returned to the hydrogenation process for recycling after dehydration and clay bed regeneration. The oxidation reaction is that the hydrogen anthraquinone in the hydrogenation liquid reacts with oxygen in the oxidation tower to form anthraquinone and hydrogen peroxide. The operating temperature is 40~60°C, the operating pressure is 0.2-0.4MPa, and the hydrogen peroxide yield can reach 95-98%. In order to adapt to the increase of production scale, the industry has continuously improved the oxidation tower to promote the uniform distribution of gas and liquid, and enhance the mass transfer of gas and liquid, such as improving the gas distributor device, changing the position of compressed air entering the tower, and adopting new packing, etc. Although these improvements overcome amplification effect to a certain extent, there are still some shortcomings such as uneven gas-liquid distribution, large bed resistance, small gas-liquid interface area, and limited mass transfer capacity.

The micro-interface reaction strengthening technology can fundamentally make the gas-liquid distribution uniform by forming a micro-interface reaction system, and it can at the same time greatly increase the gas-liquid phase interface area, enhance mass transfer, and avoid the shortage caused by fillers. A micro-interface strengthening reactor bubble scale structure-effect controlled mathematical model modeling method (Public number: CN107563051A) quantifies the relationship between the bubble scale of the reactor and the structural parameters, operating parameters and physical parameters of the reactor. When the ventilation rate is constant, the smaller the bubble size, the larger the gas-liquid interface area, the smaller the mass transfer resistance, and the larger the microscopic reaction rate. Studies by Santacesaria et al. have shown that the intrinsic speed of the reaction between 2-ethyltetrahydroanthraquinone and oxygen is a medium-slow reaction. Therefore, when the production capacity is fixed, how to determine the evaluation method of micro-bubble strengthening in the hydrogen peroxide system produced by anthraquinone method, to maximize the space-time yield and realize the economical efficiency of the micro-interface reaction strengthening technology is an important practical problem.

In view of this, the present invention is proposed.

## SUMMARY OF THE INVENTION

In view of this, the objective of the present invention is to realize the economical efficiency of the micro-interface reaction strengthening technology, determine the micro-interface reaction strengthening evaluation method for hydrogen peroxide oxidation reaction prepared by the anthraquinone method, in order to optimize the design and operation of the oxidation tower, and construct a structure-effect controlled mathematical model of the micro-interface oxidation reaction system for hydrogen peroxide produced by the anthraquinone method, thereby theoretically discussing the evaluation method of oxidation reaction micro-interface reaction strengthening.

In the actual process, the reaction rate generally refers to the macroscopic reaction rate, not the intrinsic reaction rate. The macroscopic reaction rate is controlled by mass transfer, which means that the intrinsic reaction rate is faster, but the mass transfer rate is slower, or both the intrinsic reaction rate and the mass transfer rate affect the reaction process. The study on the enhancement of mass transfer is of great significance to the enhancement of the reaction process.

The scientific and effective way to enhance gas-liquid mass transfer is to break the gas phase entering the reactor into micron-sized bubbles as much as possible, and to form a micro-interface mass transfer and reaction system in the reactor. Since there are three orders of magnitude in the micron scale, there is a question of which scale the bubbles are broken into, that is, how to grasp the "degree". From the perspective of strengthening the phase boundary area, the smaller the bubbles, the larger the phase boundary area. In general, when the bubbles in the system are large, the mass transfer rate is slower than the intrinsic reaction rate, and the reduction of the bubble size is conducive to the increase of the mass transfer rate; but at the same time, the intrinsic reaction rate decreases accordingly. Therefore, in

the process of gradually reducing the bubble size of the micro-interface reaction system, a situation is bound to appear, namely: mass transfer rate = intrinsic reaction rate. After that, the reduction of the bubble size will lead to the mass transfer rate of the system being greater than the intrinsic reaction rate, and the influence of the mass transfer rate on the macroscopic reaction rate of the system has been placed in a secondary position. Therefore, continuing to reduce the bubble size not only increases energy consumption, but also has little practical significance for increasing the macroscopic reaction rate.

Hence, in actual working conditions, bubbles are not as small as possible.

In order to achieve the above objectives, the present invention provides an evaluation method for micro-bubble strengthening in a hydrogen peroxide system prepared by anthraquinone method, including the following steps:

(a) establishing an oxidation reaction equation;

an oxidation section of the anthraquinone method includes: 2-ethyltetrahydroanthraquinone reacts with oxygen to generate hydrogen peroxide, and a chemical equation is:

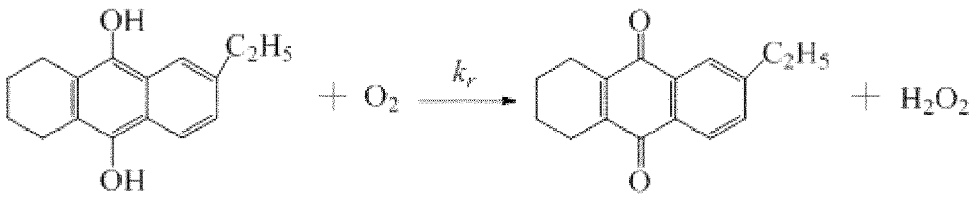


A B C D (1);

a reaction rate equation for an reaction of 2-ethyltetrahydroanthraquinone and oxygen is:

$$-r_B = k_r C_A C_B \qquad (2);$$

in the equation (2), $k_r$ is a reaction rate constant, in $m^3/(mol \cdot s)$; $C_A$ and $C_B$ are concentrations of 2-ethyltetrahydroanthraquinone and oxygen in the liquid phase, in $mol/m^3$;

(b) constructing a macroscopic reaction kinetic equation of a reaction gas;

a mass transfer rate of oxygen in a gas-liquid film is set to be equal to the reaction rate in the liquid phase during the oxidation reaction, and an equation (3) is obtained:

$$k_G a(P_B/H_B - C_{Bi}) = k_L a E_B (C_{Bi} - C_B) = k_r C_A C_B \qquad (3);$$

solving the equation (3) to obtain an equation (4):

$$-r_B = \frac{1}{\frac{1}{k_G a} + \frac{1}{k_L a E_B} + \frac{1}{k_r C_A}} \cdot \frac{P_B}{H_B} \qquad (4);$$

in the equation (4), $r_B$ is the macroscopic reaction rate of oxygen, in $mol/(m^3 \cdot s)$; $k_G$ and $k_L$ are gas film and liquid film mass transfer coefficients, respectively, in m/s; $a$ is a gas-liquid interface area, in $m^2/m^3$; $E_B$ is an reaction enhancement factor of oxygen; $P_B$ is a partial pressure of oxygen in a gas phase body in a bubble, in Pa; $H_B$ is a Henry coefficient of oxygen, in $Pa \cdot m^3/mol$;

a relationship between $k_G$, $k_L$ and $a$ in the equation (4) and a bubble diameter $d_{32}$ of the reaction system are respectively:

$$k_G = \frac{d_{32}}{6t_{32}}\left[\frac{D_{BG}\pi^2 t_{32}}{(d_{32}/2)^2} - \ln\frac{6}{\pi^2}\right] \quad (5);$$

$$k_L = 2\sqrt{D_{BL}v_s/(\pi d_{32})} \quad (6);$$

$$a = \frac{6v_G}{d_{32}v_{32}} \quad (7);$$

in the equation (5), $t_{32}$ is a residence time of bubbles, in s; if an operating liquid level of the reactor is $H_0$, then:

$$t_{32} = H_0/v_{32} \quad (8);$$

in the equation (6), $v_s$ is a bubble slip velocity, in m/s, and a calculation equation is:

$$v_s = \left|v_{32} - \frac{v_L}{1 - v_G/v_{32}}\right| \quad (9);$$

in the equations (7) ~ (9), $v_{32}$ is a rising speed of a bubble group, in m/s, and a calculation equation is:

$$v_{32} = \frac{(v_0 + v_G + v_L) + \sqrt{(v_0 + v_G + v_L)^2 - 4v_0 v_G}}{2} \quad (10);$$

in the equation (10), $v_0$ is a rising speed of a single bubble in the infinite static liquid, in m/s, and a calculation equation is:

$$v_0 = \left(\frac{\sigma_L g}{\rho_L}\right)^{1/4}\left[\left(\frac{Mo^{-1/4}}{K_b}d_e^2\right)^{-n} + \left(\frac{2c}{d_e} + \frac{d_e}{2}\right)^{-n/2}\right]^{-1/n} \quad (11);$$

in the equation (11), $Mo$ is a Morton number, which is a dimensionless parameter that describes a shape of the bubble when it moves in the liquid phase, $Mo = g\mu L^4/(\rho_L\sigma_L^3)$; $d_e$ is the dimensionless bubble diameter, $d_e = d_{32}(p_L g/\sigma_L)^{1/2}$ ; $K_b$ is a constant related to physical properties of the system, $K_b = K_{b0} Mo^{-0.038}$, for organic solvents or mixtures, $K_{b0}$ =10.2, if $K_b$ <12, then taking $K_b$ =12 for calculation; constants $c$ and $n$ take corresponding empirical values according to characteristics of the actual system;

(c) constructing a mathematical expression of the reaction enhancement factor $E_B$ of oxygen;

a $Ha$ number of the reaction system in which 2-ethyltetrahydroanthraquinone reacts with oxygen to generate hydrogen peroxide is:

$$Ha = \frac{\sqrt{k_r C_A D_{BL}}}{k_L} \quad (12);$$

the $Ha$ number is an abbreviation of Hatta, which is a dimensionless parameter, and the Hatta number represents

a relative size of a maximum possible conversion rate and the maximum mass transfer rate of reaction gas molecules in the liquid film, which indicates a relative strength of reaction and mass transfer;

$E_\infty$ is expressed by:

$$E_\infty = 1 + \frac{D_{AL} C_A}{D_{BL} C_{Bi}} \quad (13);$$

$E_\infty$ represents the enhancements of mass transfer by the liquid-phase main reaction far away from the gas-liquid interface;

then the expression of the response enhancement factor $E_B$ is:

$$E_B = \left( Ha \sqrt{\frac{E_\infty - E_B}{E_\infty - 1}} \right) \Big/ \tanh\left( Ha \sqrt{\frac{E_\infty - E_B}{E_\infty - 1}} \right) \quad (14);$$

(d) constructing a calculation method for the concentration of each reaction component in the liquid phase, and calculating the concentration of each reaction component in the liquid phase;

setting the reactor as plug-flow reactor, then for components 2-ethyltetrahydroanthraquinone A and oxygen B, equations (15) - (16) are obtained:

$$\frac{dC_A}{dH_r} = \frac{S_r}{Q_L} k_r C_A C_B \quad (15);$$

$$\frac{dF_B}{dH_r} = S_r k_r C_A C_B \quad (16);$$

obtaining an equation (17) according to both the partial pressures of oxygen and nitrogen I in the gas phase and the relation of molar flow:

$$\frac{F_B}{F_I + F_B} = \frac{P_B}{P_T} \quad (17);$$

the concentration $C_B$ at an inlet is obtained according to the $F_B$ and $C_A$ at the inlet and the simultaneous equations (3), (5) - (14) and (17), and the concentration $C_A$ and the concentration $C_B$ in the liquid phase at any position in the axial direction are obtained according to the equations (15) and (16).

Further, before the step (a) for establishing the oxidation reaction equation, the evaluation method further includes: a hydrogenation section of the anthraquinone method, wherein the hydrogenation section reacts to generate the hydrogenation liquid.

Further, the reaction product in the hydrogenation liquid includes one or more of the following: hydroanthraquinone, tetrahydroanthraquinone, 2-ethylhydroanthraquinone, and 2-ethyltetrahydroanthraquinone.

Further, the oxidation section of the anthraquinone method further includes one or more of the following: hydrogen anthraquinone reacting with oxygen to generate hydrogen peroxide, tetrahydroanthraquinone reacting with oxygen to generate hydrogen peroxide, and 2-ethylhydroanthraquinone reacting with oxygen to generate hydrogen peroxide.

In the evaluation method of the present invention, firstly, by constructing the macroscopic kinetic equation of

the reaction gas, and then by constructing the mathematical expression of the reaction enhancement factor $E_B$ of oxygen, a calculation method for the concentration of each reaction component in the liquid phase is constructed. The concentration of the reaction components and the macroscopic reaction rate are calculated, and then the bubble size in the reaction system is compared with the concentration of the corresponding reaction components and the macroscopic reaction rate to obtain the optimal bubble size of the reaction system and evaluate the effect of the reaction enhancement.

Through many experiments, it's found that only when the bubble size just satisfies the mass transfer rate of the reaction gas being equal to the consumption rate of the reaction gas in the liquid phase, the macro-interface strengthening reaction state is optimal. Then $X_A$, $r_A$ and $C_A$ are calculated according to the determined bubble size. How to determine the bubble size has been involved in previous patents and belongs to the prior art. The key improvement point of the present invention is how to determine the most suitable range of the bubble size in the micro-interface reaction system of hydrogen peroxide produced by anthraquinone method.

For related modeling method of micro-interface strengthening reactor bubble scale, many previous patents are also involved, such as patent publication numbers CN109684769A, CN107346378A, CN107335390A, and CN107561938A, etc. Parameters such as macroscopic reaction rate $r_B$ and mass transfer coefficient $K_G$ involved in the macroscopic reaction kinetic equation established by adopting the evaluation method of the present invention and many other calculation formulas all have certain relevance with $d_{32}$, however, the relevant formulas belong to the prior art and have been involved in the previous patents, such as the formulas (3), (5) ~(14) and (17) listed in the present invention. The present invention is re-innovated based on many previous patents to explore the influence of the bubble size on the actual working conditions in the anthraquinone hydrogen peroxide system, such as the calculation methods of $r_B$, $E_B$ and $C_B$, etc. These parameters can well reflect the relationship between these parameters and the bubble size under actual working conditions, and these calculation methods are not involved in previous patents.

The present invention also provides a micro-interface reaction strengthening reactor, which is designed by adopting the above-mentioned evaluation method for strengthening the micro-bubbles in the hydrogen peroxide system produced by the anthraquinone method. To optimize the design and operation of the micro-interface strengthening reactor by discussing the most suitable bubble size in the hydrogen peroxide system produced by the anthraquinone method. The micro-interface strengthening reactor designed by the above calculation method is more in line with the actual application situation, and the bubble size can be controlled to the most ideal state, so that the micro-interface strengthening reactor can achieve excellent reaction effect.

In addition, the reactor structure of the present invention can be referred to the patent CN106187660A previously applied by the inventor, which is not repeated in the present invention. The present invention designs related reactor structure parameters based on the constructed structure-effect controlled mathematical model and the associated influence of operating parameters on the reaction gas transmission effect.

Compared with prior art, the beneficial effect of the present invention is:

The present invention discusses the effect of bubble size on gas-liquid phase interface area, volumetric mass transfer coefficient and macroscopic reaction rate by constructing a structure-effect controlled mathematical model of anthraquinone-based hydrogen peroxide oxidation reaction system and evaluates the strengthening effect of micro-interface oxidation reaction, to provide a basis for the design and operation of micro-interface reaction strengthening reactors. The reactor designed by the evaluation method of the present invention can solve the problem of uneven gas-liquid distribution and enhance mass transfer at the same time, thereby maximizing the space-time yield and realizing the economy of micro-interface reaction strengthening technology.

## BRIEF DESCRIPTION OF THE DRAWINGS

Various other advantages and benefits will become apparent to those of ordinary skill in the art upon reading the following detailed description of the preferred embodiments. The drawings are merely for the purpose of illustrating preferred embodiments and are not to be considered as limiting the invention. In the attached drawings:

Fig. 1 is a graph showing the influence of the bubble size $d_{32}$ on the gas-liquid phase interface area *a* according to an embodiment of the present invention;

Fig. 2 is a graph showing the influence of the bubble size $d_{32}$ on the gas side volumetric mass transfer coefficient $k_G$ and the liquid side volumetric mass transfer coefficient $k_L$ according to an embodiment of the present invention;

Fig. 3 is a graph of the concentration $C_A$ of 2-ethyltetrahydroanthraquinone at different axial heights under different bubble sizes $d_{32}$ according to an embodiment of the present invention;

Fig. 4 is a graph of the concentration $C_B$ of $O_2$ in the liquid phase at different axial heights under different bubble sizes $d_{32}$ according to an embodiment of the present invention; and

Fig. 5 is a graph showing the influence of the bubble size $d_{32}$ on the macroscopic reaction rate $R_{macro}$ according to an embodiment of the present invention.

## DESCRIPTION OF THE PREFERRED EMBODIMENTS

Exemplary embodiments will be described in detail herein, and examples of which are illustrated in the accompanying drawings. When the following description refers to the accompanying drawings, the same numerals in different drawings refer to the same or similar elements unless otherwise indicated. The implementations described in the following exemplary embodiments do not represent all implementations consistent with the present invention. Rather, they are merely examples of apparatuses and methods consistent with aspects of the present invention are recited in the appended claims.

The terminologies used in the present invention are for the purpose of describing embodiments only and are not intended to limit the present invention. As used in the invention and the appended claims, the singular forms "a" and "said" and "the" are intended to include the plural forms as well, unless the context clearly dictates otherwise. It should also be understood that the term "and/or" as used herein refers to and includes any and all possible combinations of one or more of the associated listed items.

It should be understood that although the terms first, second, third, etc. may be used in the present invention to describe various information, the information should not be limited to these terms. These terms are only used to distinguish information of the same type from one another. For example, without departing from the scope of the present invention, first information may also be called second information, and similarly, second information may also be called first information. Depending on the context, the word "if' as used herein may be interpreted as "at" or "when" or "in response to a determination".

In order to illustrate the technical schemes in the present invention more clearly, the following will be described in the form of specific embodiments.

### Embodiments

This embodiment is based on the evaluation method of the present invention, aiming at the bubbling reactor of the hydrogen peroxide oxidation section of an anthraquinone process in a certain enterprise and the existing operating conditions, the effect of the bubble size in the reactor on the gas-liquid phase interface area, gas-liquid mass transfer coefficient and influence of macroscopic reaction rate of 2-ethyltetrahydroanthraquinone.

The calculation conditions are as follows:
The operating parameters of the reactor:

the height of the operating liquid level of the original rector $H_0$=11.3m;

the cross-sectional area of the reactor $S_0$=0.1256m$^2$;

the liquid phase density =$\rho_L$=906kg/m$^3$;

the viscosity $\mu_L$=1.22mPa • s;

the surface tension $\sigma_L$=27.02mN/m;

the operating pressure $P_T$=2.25bar (A);

the operating temperature T=323.15K;

the liquid flow $Q_L$=4.57 × 10-4m$^3$/s ;

the air flow $Q_G$=0.0172Nm$^3$/s;

the 2-Ethyltetrahydroanthraquinone molar flow $F_A$=0.439mol/h.

The evaluation method for micro-interface reaction strengthening in a hydrogen peroxide system prepared by anthraquinone method according to the embodiment of the present invention specifically includes the following steps:

(a) establishing an oxidation reaction equation;

the reactants in the hydrogenation solution include one or more of hydroanthraquinone, tetrahydroanthraquinone, 2-ethylhydroanthraquinone and 2-ethyltetrahydroanthraquinone. Taking 2-ethyltetrahydroanthraquinone as an example, the chemical equation for the reaction of 2-ethyltetrahydroanthraquinone with oxygen to form hydrogen peroxide is:

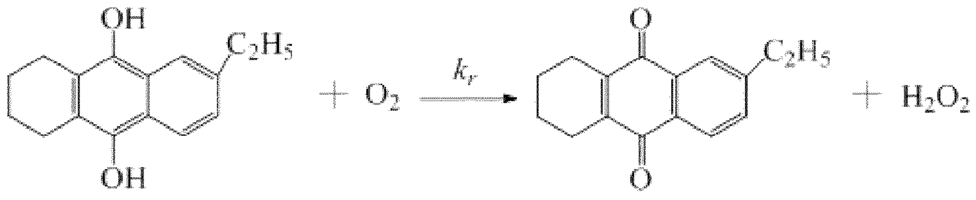


A B C D (1).

The reaction between 2-ethyltetrahydroanthraquinone and oxygen is a second-order reaction, and its reaction rate equation is:

$$-r_B = k_r C_A C_B \qquad (2);$$

in the equation (2), $k_r$ is a reaction rate constant, in m$^3$/(mol·s); $C_A$ and $C_B$ are concentrations of 2-ethyltetrahydroanthraquinone and oxygen in the liquid phase, in mol/m$^3$;

(b) constructing a macroscopic reaction kinetic equation of a reaction gas;

the mass transfer rate of oxygen in a gas-liquid film is set to be equal to the reaction rate in the liquid phase during the oxidation reaction, and an equation (3) is obtained:

$$k_G a(P_B/H_B - C_{Bi}) = k_L a E_B (C_{Bi} - C_B) = k_r C_A C_B \qquad (3);$$

solving the equation (3) to obtain an equation (4):

$$-r_B = \frac{1}{\frac{1}{k_G a} + \frac{1}{k_L a E_B} + \frac{1}{k_r C_A}} \cdot \frac{P_B}{H_B} \qquad (4);$$

in the equation (4), $r_B$ is the macroscopic reaction rate of oxygen, in mol/(m$^3$ • s); $k_G$ and $k_L$ are gas film and liquid film mass transfer coefficients, respectively, in m/s; $a$ is a gas-liquid interface area, in m$^2$/m$^3$; $E_B$ is an reaction enhancement factor of oxygen; $P_B$ is a partial pressure of oxygen in a gas phase body in a bubble, in Pa; $H_B$ is a Henry coefficient of oxygen, in Pa • m$^3$/mol.

The corresponding structure-effect controlled mathematical models of the $k_G$, $k_L$ and $a$ in the equation (4) have been established, and their relationships between a bubble diameter $d_{32}$ of the reaction system are respectively:

$$k_G = \frac{d_{32}}{6t_{32}}\left[\frac{D_{BG}\pi^2 t_{32}}{(d_{32}/2)^2} - \ln\frac{6}{\pi^2}\right] \qquad (5);$$

$$k_L = 2\sqrt{D_{BL} v_s/(\pi d_{32})} \qquad (6);$$

$$a = \frac{6v_G}{d_{32}v_{32}} \quad (7);$$

in the equation (5), $t_{32}$ is a residence time of bubbles, in s; if an operating liquid level of the reactor is $H_0$, then:

$$t_{32} = H_0/v_{32} \quad (8);$$

in the equation (6), $v_s$ is a bubble slip velocity, in m/s, and a calculation equation is:

$$v_s = \left| v_{32} - \frac{v_L}{1 - v_G/v_{32}} \right| \quad (9);$$

in the equations (7) ~ (9), $v_{32}$ is a rising speed of a bubble group, in m/s, and a calculation equation is:

$$v_{32} = \frac{(v_0 + v_G + v_L) + \sqrt{(v_0 + v_G + v_L)^2 - 4v_0v_G}}{2} \quad (10);$$

in the equation (10), $v_0$ is a rising speed of a single bubble in the infinite static liquid, in m/s, and a calculation equation is:

$$v_0 = \left(\frac{\sigma_L g}{\rho_L}\right)^{1/4} \left[ \left(\frac{Mo^{-1/4}}{K_b} d_e^2\right)^{-n} + \left(\frac{2c}{d_e} + \frac{d_e}{2}\right)^{-n/2} \right]^{-1/n} \quad (11);$$

in the equation (11), $Mo$ is a Morton number, which is a dimensionless parameter that describes a shape of the bubble when it moves in the liquid phase, $Mo = g\mu_L^4/(\rho_L\sigma_L^3)$, $d_e$ is the dimensionless bubble diameter, $d_e = d_{32}(\rho_L g/\sigma_L)^{1/2}$ ; $K_b$ is a constant related to physical properties of the system, $K_b = K_{b0} Mo^{-0.038}$, for organic solvents or mixtures, $K_{b0}$ =10.2, if $K_b$ <12, then taking $K_b$ =12 for calculation; constants c and $n$ take corresponding empirical values according to characteristics of the actual system.

(c) constructing a mathematical expression of the reaction enhancement factor $E_B$ of oxygen;

a $Ha$ number of the reaction system in which 2-ethyltetrahydroanthraquinone reacts with oxygen to generate hydrogen peroxide is:

$$Ha = \frac{\sqrt{k_r C_A D_{BL}}}{k_L} \quad (12);$$

the $Ha$ number is an abbreviation of Hatta, which is a dimensionless parameter, and the Hatta number represents a relative size of a maximum possible conversion rate and the maximum mass transfer rate of reaction gas molecules in the liquid film, which indicates a relative strength of reaction and mass transfer;

$E_\infty$ is expressed by:

$$E_\infty = 1 + \frac{D_{AL}C_A}{D_{BL}C_{Bi}} \quad (13);$$

$E_\infty$ represents the enhancements of mass transfer by the liquid-phase main reaction far away from the gas-liquid interface;

then the expression of the response enhancement factor $E_B$ is:

$$E_B = \left(Ha\sqrt{\frac{E_\infty - E_B}{E_\infty - 1}}\right) \Big/ \tanh\left(Ha\sqrt{\frac{E_\infty - E_B}{E_\infty - 1}}\right) \quad (14);$$

(d) constructing a calculation method for the concentration of each reaction component in the liquid phase, and calculating the concentration of each reaction component in the liquid phase;

setting the reactor as plug-flow reactor, then for components 2-ethyltetrahydroanthraquinone A and oxygen B, equations (15) ~ (16) are obtained:

$$\frac{dC_A}{dH_r} = \frac{S_r}{Q_L} k_r C_A C_B \quad (15);$$

$$\frac{dF_B}{dH_r} = S_r k_r C_A C_B \quad (16);$$

obtaining an equation (17) according to both the partial pressures of oxygen and nitrogen I in the gas phase and the relation of molar flow:

$$\frac{F_B}{F_I + F_B} = \frac{P_B}{P_T} \quad (17);$$

the concentration $C_B$ at an inlet is obtained according to the $F_B$ and $C_A$ at the inlet and the simultaneous equations (3), (5) - (14) and (17). The concentration $C_A$ and the concentration $C_B$ in the liquid phase at any position in the axial direction are obtained according to the equations (15) and (16).

In addition to the varying relationship between the macroscopic reaction rate and the concentration of oxygen and 2-ethyltetrahydroanthraquinone in the liquid phase at any position in the axial direction and the bubble size, the varying relationship between gas-liquid phase interface area and the volumetric mass transfer coefficient of the gas and liquid sides and the bubble size can also be obtained. The specific varying relationship is shown in Fig. 1 to Fig. 5.

Fig. 1 shows the influence of the bubble size $d_{32}$ on the gas-liquid phase interface area *a*. As can be seen from Fig. 1, the gas-liquid phase interface area *a* increases continuously with the decrease of the bubble size $d_{32}$ in the reaction system. In this embodiment, the bubble size $d_{32}$ is reduced from 8.4 mm to 0.2 mm, and the gas-liquid phase interface area is increased from 168m$^2$/m$^3$ to 26617 m$^2$/m$^3$, which is an increase of about 157 times.

Fig. 2 shows the influence of the bubble size $d_{32}$ on the gas side volumetric mass transfer coefficient $k_G$ and the liquid side volumetric mass transfer coefficient $k_L$. As can be seen from Fig. 2, both the gas side volumetric mass transfer coefficient $k_G$ and the liquid side volumetric mass transfer coefficient $k_L$ increase with the decrease of the bubble size $d_{32}$.

Fig. 3 shows a variation of the concentration $C_A$ of 2-ethyltetrahydroanthraquinone in the axial direction under different conditions of bubble sizes $d_{32}$. As can be seen from Fig. 3, the smaller the bubble size $d_{32}$, the faster the concentration $C_A$ of 2-ethyltetrahydroanthraquinone in the axial direction decreases, that is, the smaller the bubble size

$d_{32}$, the faster the reaction rate. At the same time, to achieve the same conversion rate, the required liquid level decreases as the bubble size $d_{32}$ decreases. In this embodiment, the bubble size $d_{32}$ is decreased from 8.4 mm to 0.5 mm, the required liquid level height is reduced from 11.3m to 5.5m for achieving the same conversion rate.

Fig. 4 shows a variation of the axial concentration $C_B$ of $O_2$ in the liquid phase under different conditions of bubble sizes $d_{32}$. As can be seen from Fig. 4, due to the influence of mass transfer and reaction, the axial concentration variations of $O_2$ in the liquid phase are not consistent under different conditions of bubble sizes $d_{32}$. However, since the volumetric mass transfer coefficients of the gas and liquid sides increase with the decrease of the bubble size $d_{32}$, the overall concentration of $O_2$ in the liquid phase increases with the decrease of the bubble size $d_{32}$.

Fig. 5 shows the influence of the bubble size $d_{32}$ on the macroscopic reaction rate $R_{macro}$. As can be seen from Fig. 5, the macroscopic reaction rate $R_{macro}$ increases with the decrease of the bubble size $d_{32}$. In this embodiment, the bubble size $d_{32}$ was reduced from 8.4 mm to 0.2 mm, and the macroscopic reaction rate $R_{macro}$ was increased from 0.0105mol/($m^3$·s) to 0.0216 mol/($m^3$ ·s), which is enhanced by about 106%.

The technical schemes of the present invention are described in conjunction with the preferred embodiments shown in the accompanying drawings, however, those skilled in the art will easily understand that the protection scope of the present invention is obviously not limited to these specific embodiments. Without departing from the principles of the present invention, those skilled in the art can make equivalent modifications or substitutions to related technical features, and the technical schemes after these modifications or substitutions will all fall within the protection scope of the present invention.

The above descriptions are merely preferred embodiments of the present invention and are not intended to limit the present invention. Any modifications, equivalent replacements, improvements, etc. made within the spirit and principles of the present invention shall be included in the present invention within the scope of protection.

## Claims

1. An evaluation method for micro-bubble strengthening in a hydrogen peroxide system prepared by anthraquinone method, **characterized in that** it comprises the following steps:

   (a) establishing an oxidation reaction equation;

   an oxidation section of the anthraquinone method includes: 2-ethyltetrahydroanthraquinone reacts with oxygen to generate hydrogen peroxide, and a chemical equation is:

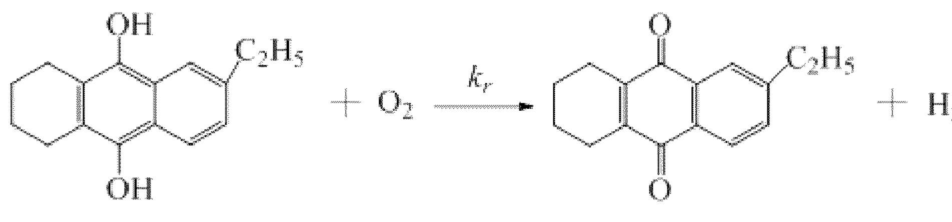

A B C D (1);

   a reaction rate equation for an reaction of 2-ethyltetrahydroanthraquinone and oxygen is:

$$-r_B = k_r C_A C_B \qquad (2);$$

   in the equation (2), $k_r$ is a reaction rate constant, in $m^3$/(mol·s); $C_A$ and $C_B$ are concentrations of 2-ethyltetrahydroanthraquinone and oxygen in the liquid phase, in mol/$m^3$;

   (b) constructing a macroscopic reaction kinetic equation of a reaction gas;

   a mass transfer rate of oxygen in a gas-liquid film is set to be equal to the reaction rate in the liquid phase during the oxidation reaction, and an equation (3) is obtained:

$$k_G a(P_B/H_B - C_{Bi}) = k_L a E_B (C_{Bi} - C_B) = k_r C_A C_B \qquad (3);$$

solving the equation (3) to obtain an equation (4):

$$-r_B = \frac{1}{\frac{1}{k_G a} + \frac{1}{k_L a E_B} + \frac{1}{k_r C_A}} \cdot \frac{P_B}{H_B} \quad (4);$$

in the equation (4), $r_B$ is the macroscopic reaction rate of oxygen, in mol/(m$^3$ • s); $k_G$ and $k_L$ are gas film and liquid film mass transfer coefficients, respectively, in m/s; $a$ is a gas-liquid interface area, in m$^2$/m$^3$; $E_B$ is an reaction enhancement factor of oxygen; $P_B$ is a partial pressure of oxygen in a gas phase body in a bubble, in Pa; $H_B$ is a Henry coefficient of oxygen, in Pa • m$^3$/mol;
a relationship between *kc*, $k_L$ and *a* in the equation (4) and a bubble diameter $d_{32}$ of the reaction system are respectively:

$$k_G = \frac{d_{32}}{6t_{32}}\left[\frac{D_{BG}\pi^2 t_{32}}{(d_{32}/2)^2} - \ln\frac{6}{\pi^2}\right] \quad (5);$$

$$k_L = 2\sqrt{D_{BL} v_s/(\pi d_{32})} \quad (6);$$

$$a = \frac{6v_G}{d_{32} v_{32}} \quad (7);$$

in the equation (5), $t_{32}$ is a residence time of bubbles, in s; if an operating liquid level of the reactor is $H_0$, then:

$$t_{32} = H_0/v_{32} \quad (8);$$

in the equation (6), $v_s$ is a bubble slip velocity, in m/s, and a calculation equation is:

$$v_s = \left| v_{32} - \frac{v_L}{1 - v_G/v_{32}} \right| \quad (9);$$

in the equations (7) ~ (9), $v_{32}$ is a rising speed of a bubble group, in m/s, and a calculation equation is:

$$v_{32} = \frac{(v_0 + v_G + v_L) + \sqrt{(v_0 + v_G + v_L)^2 - 4v_0 v_G}}{2} \quad (10);$$

in the equation (10), $v_0$ is a rising speed of a single bubble in the infinite static liquid, in m/s, and a calculation equation is:

$$v_0 = \left(\frac{\sigma_L g}{\rho_L}\right)^{1/4}\left[\left(\frac{Mo^{-1/4}}{K_b} d_e^2\right)^{-n} + \left(\frac{2c}{d_e} + \frac{d_e}{2}\right)^{-n/2}\right]^{-1/n} \quad (11);$$

in the equation (11), *Mo* is a Morton number, which is a dimensionless parameter that describes a shape of the bubble when it moves in the liquid phase, $Mo = g\mu_L^4/(\rho_L \sigma_L^3)$; $d_e$ is the dimensionless bubble diameter, $d_e$ - $d_{32}$ $(p_L\ g/\sigma_L)^{1/2}$ ; $K_b$ is a constant related to physical properties of the system, $K_b = K_{b0}\ Mo^{-0.038}$, for

organic solvents or mixtures, $K_{b0}$ =10.2, if $K_b$ <12, then taking $K_b$ =12 for calculation; constants c and $n$ take corresponding empirical values according to characteristics of the actual system;

(c) constructing a mathematical expression of the reaction enhancement factor $E_B$ of oxygen;

a *Ha* number of the reaction system in which 2-ethyltetrahydroanthraquinone reacts with oxygen to generate hydrogen peroxide is:

$$Ha = \frac{\sqrt{k_r C_A D_{BL}}}{k_L} \quad (12);$$

the *Ha* number is an abbreviation of Hatta, which is a dimensionless parameter, and the Hatta number represents a relative size of a maximum possible conversion rate and the maximum mass transfer rate of reaction gas molecules in the liquid film, which indicates a relative strength of reaction and mass transfer; $E_\infty$ is expressed by:

$$E_\infty = 1 + \frac{D_{AL} C_A}{D_{BL} C_{Bi}} \quad (13);$$

$E_\infty$ represents the enhancements of mass transfer by the liquid-phase main reaction far away from the gas-liquid interface;
then the expression of the response enhancement factor $E_B$ is:

$$E_B = \left( Ha\sqrt{\frac{E_\infty - E_B}{E_\infty - 1}} \right) \Big/ \tanh\left( Ha\sqrt{\frac{E_\infty - E_B}{E_\infty - 1}} \right) \quad (14);$$

(d) constructing a calculation method for the concentration of each reaction component in the liquid phase, and calculating the concentration of each reaction component in the liquid phase;

setting the reactor as plug-flow reactor, then for components 2-ethyltetrahydroanthraquinone A and oxygen B, equations (15) - (16) are obtained:

$$\frac{dC_A}{dH_r} = \frac{S_r}{Q_L} k_r C_A C_B \quad (15);$$

$$\frac{dF_B}{dH_r} = S_r k_r C_A C_B \quad (16);$$

obtaining an equation (17) according to both the partial pressures of oxygen and nitrogen I in the gas phase and the relation of molar flow:

$$\frac{F_B}{F_I + F_B} = \frac{P_B}{P_T} \quad (17);$$

the concentration $C_B$ at an inlet is obtained according to the $F_B$ and $C_A$ at the inlet and the simultaneous

equations (3), (5) - (14) and (17), and the concentration $C_A$ and the concentration $C_B$ in the liquid phase at any position in the axial direction are obtained according to the equations (15) and (16).

2. The evaluation method for micro-bubble strengthening in a hydrogen peroxide system prepared by anthraquinone method according to claim 1, **characterized in that,** before the step (a) for establishing the oxidation reaction equation, it further comprises: a hydrogenation section of the anthraquinone method, wherein the hydrogenation section reacts to generate the hydrogenation liquid.

3. The evaluation method for micro-bubble strengthening in a hydrogen peroxide system prepared by anthraquinone method according to claim 2, **characterized in that,** the reaction product in the hydrogenation liquid comprises one or more of the following: hydroanthraquinone, tetrahydroanthraquinone, 2-ethylhydroanthraquinone, and 2-ethyltetrahydroanthraquinone.

4. The evaluation method for micro-bubble strengthening in a hydrogen peroxide system prepared by anthraquinone method according to claim 3, **characterized in that,** the oxidation section of the anthraquinone method further comprises: one or more of the following: hydrogen anthraquinone reacting with oxygen to generate hydrogen peroxide, tetrahydroanthraquinone reacting with oxygen to generate hydrogen peroxide, and 2-ethylhydroanthraquinone reacting with oxygen to generate hydrogen peroxide.

5. A micro-interface reaction strengthening reactor, **characterize in that,** it is designed and obtained by adopting the evaluation method for micro-bubble strengthening in a hydrogen peroxide system prepared by anthraquinone method according to any one of claims 1-4.

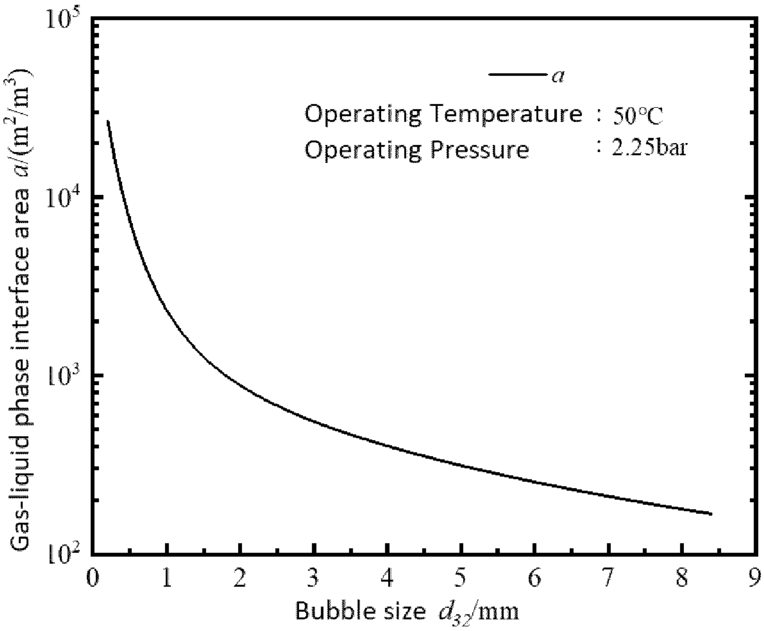

Gas-liquid phase interface area a/(m²/m³)
a
Operating Temperature : 50°C
Operating Pressure : 2.25bar
10⁵
10⁴
10³
10²
0
1
2
3
4
5
6
7
8
9
Bubble size d32/mm


Fig. 1

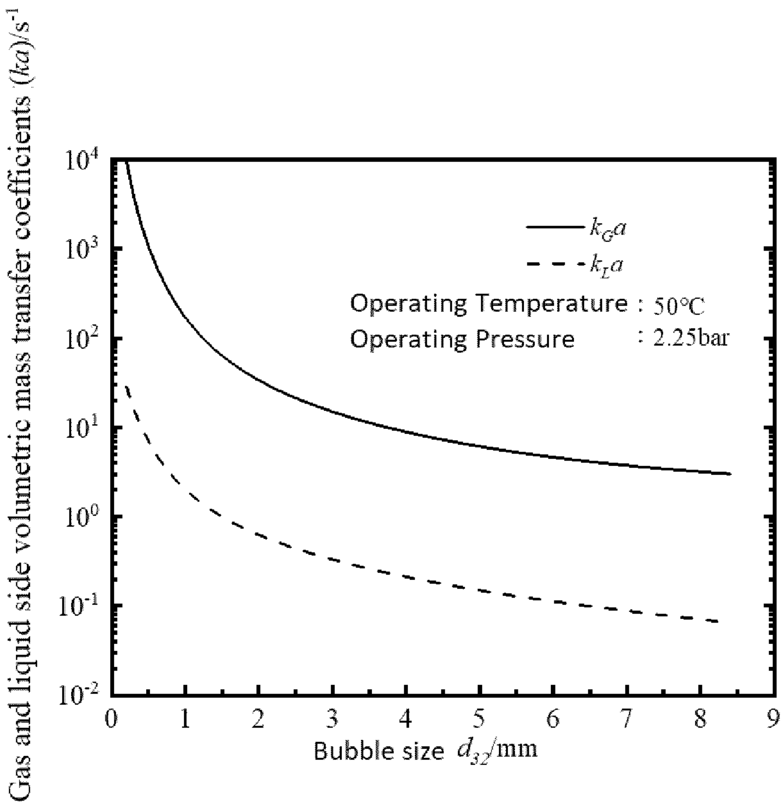

Gas and liquid side volumetric mass transfer coefficients (ka)/s-1
10-2
10-1
100
101
102
103
104
0
1
2
3
4
5
6
7
8
9
Bubble size d32/mm
kGa
kLa
Operating Temperature : 50°C
Operating Pressure : 2.25bar


Fig. 2

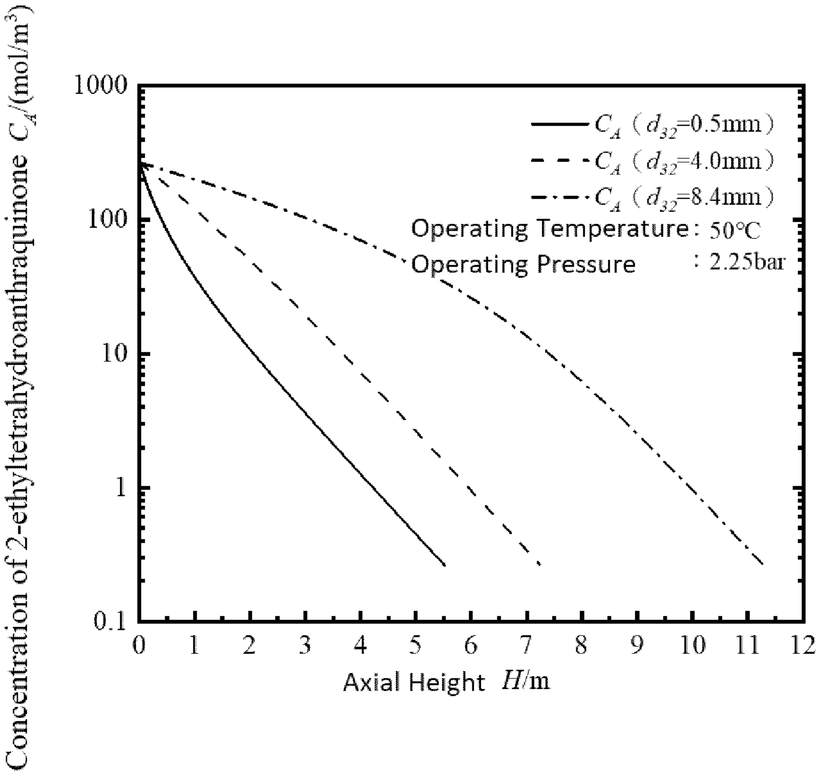

Concentration of 2-ethyltetrahydroanthraquinone $C_A$/(mol/m$^3$)
1000
100
10
1
0.1
0 1 2 3 4 5 6 7 8 9 10 11 12
Axial Height $H$/m
$C_A$ ($d_{32}$=0.5mm)
$C_A$ ($d_{32}$=4.0mm)
$C_A$ ($d_{32}$=8.4mm)
Operating Temperature: 50℃
Operating Pressure : 2.25bar


Fig. 3

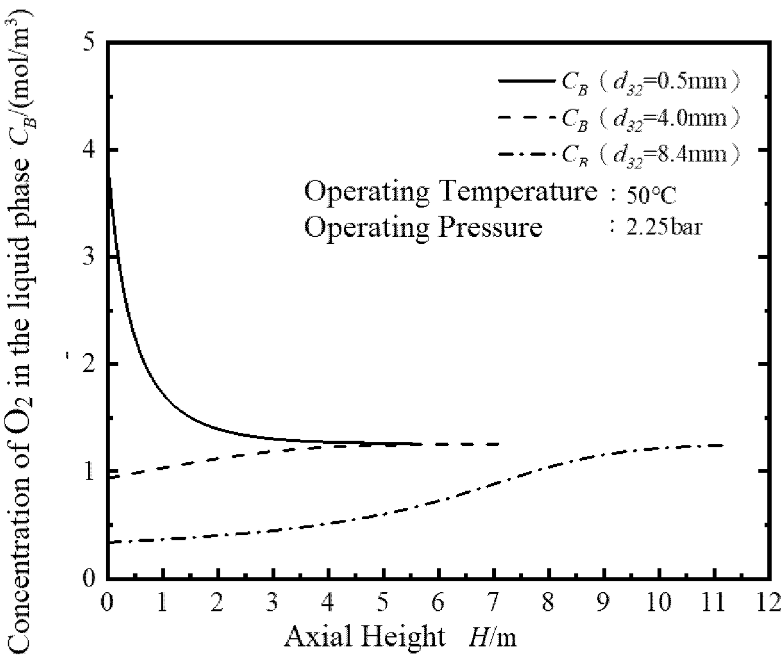

Concentration of O2 in the liquid phase CB/(mol/m3)
CB (d32=0.5mm)
CB (d32=4.0mm)
CR (d32=8.4mm)
Operating Temperature : 50°C
Operating Pressure : 2.25bar
0
1
2
3
4
5
0 1 2 3 4 5 6 7 8 9 10 11 12
Axial Height H/m


Fig. 4

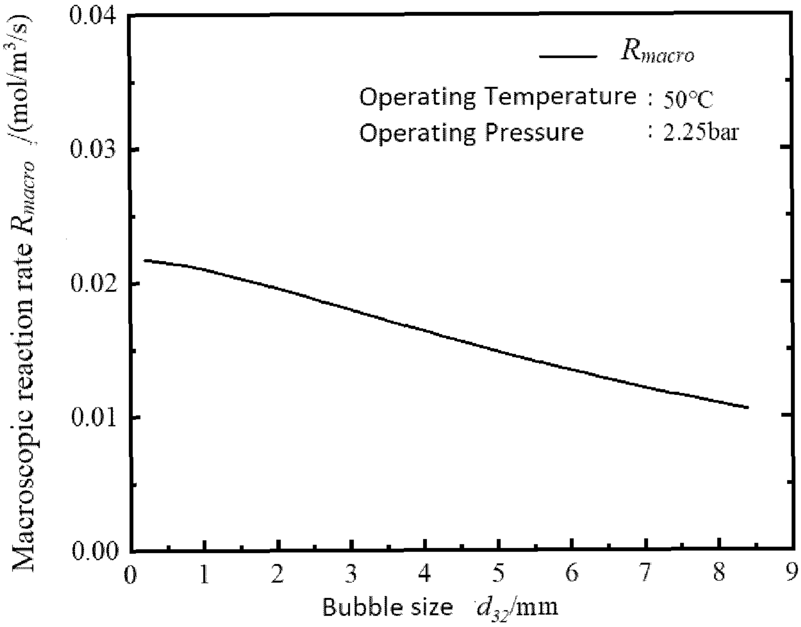
0.04
0.03
0.02
0.01
0.00
0
1
2
3
4
5
6
7
8
9
$R_{macro}$
Operating Temperature : 50°C
Operating Pressure : 2.25bar
Macroscopic reaction rate $R_{macro}$ /(mol/m³/s)
Bubble size $d_{32}$/mm

Fig. 5

**INTERNATIONAL SEARCH REPORT**

International application No.
**PCT/CN2022/103300**

**A. CLASSIFICATION OF SUBJECT MATTER**

G01N 33/00(2006.01)i；C01B 15/023(2006.01)i

According to International Patent Classification (IPC) or to both national classification and IPC

**B. FIELDS SEARCHED**

Minimum documentation searched (classification system followed by classification symbols)

G01N，C01B

Documentation searched other than minimum documentation to the extent that such documents are included in the fields searched

Electronic data base consulted during the international search (name of data base and, where practicable, search terms used)

CNTXT; CNABS; WPABS; CJFD; DWPI; ISI Web of Science: 延长反应技术研究院, 李夏冰, 李永强, 周政, 吕文钧, 田洪舟, 张锋, 张志炳, 李磊, 魏格林, 双氧水, 过氧化氢, h2o2, 蒽醌, 气泡, 传质, bubble?, microbubble?, transfer , Anthraquinone , Hydrogen peroxide

**C. DOCUMENTS CONSIDERED TO BE RELEVANT**

| Category* | Citation of document, with indication, where appropriate, of the relevant passages | Relevant to claim No. |
|---|---|---|
| Y | CN 113479850 A (NANJING YANCHANG REACTION TECHNOLOGY RESEARCH INSTITUTE CO., LTD.) 08 October 2021 (2021-10-08) description, paragraphs [0006]-[0031] | 1-5 |
| Y | CN 113066535 A (NANJING YANCHANG REACTION TECHNOLOGY RESEARCH INSTITUTE CO., LTD.) 02 July 2021 (2021-07-02) description, paragraphs [0005]-[0067] | 1-5 |
| PX | CN 114019107 A (NANJING YANCHANG REACTION TECHNOLOGY RESEARCH INSTITUTE CO., LTD.) 08 February 2022 (2022-02-08) claims 1-5 | 1-5 |
| A | CN 107032303 A (YANGZHOU HUITONG CHEMICAL ENGINEERING TECHNIQUE CO., LTD.) 11 August 2017 (2017-08-11) entire document | 1-5 |
| A | CN 107563051 A (NANJING UNIVERSITY) 09 January 2018 (2018-01-09) entire document | 1-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2022** | **26 September 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/103300**

**C. DOCUMENTS CONSIDERED TO BE RELEVANT**

| Category* | Citation of document, with indication, where appropriate, of the relevant passages | Relevant to claim No. |
|---|---|---|
| A | CN 101229915 A (CHINA UNIVERSITY OF PETROLEUM-BEIJING) 30 July 2008 (2008-07-30)<br>entire document | 1-5 |
| A | WO 0132557 A1 (DEGUSSA) 10 May 2001 (2001-05-10)<br>entire document | 1-5 |

Form PCT/ISA/210 (second sheet) (January 2015)

**INTERNATIONAL SEARCH REPORT**
**Information on patent family members**

International application No.
**PCT/CN2022/103300**

| Patent document cited in search report | Publication date (day/month/year) | Patent family member(s) | Publication date (day/month/year) |
|---|---|---|---|
| CN 113479850 A | 08 October 2021 | None | |
| CN 113066535 A | 02 July 2021 | None | |
| CN 114019107 A | 08 February 2022 | None | |
| CN 107032303 A | 11 August 2017 | None | |
| CN 107563051 A | 09 January 2018 | None | |
| CN 101229915 A | 30 July 2008 | None | |
| WO 0132557 A1 | 10 May 2001 | DE 50010007 D1 | 12 May 2005 |
| | | AU 1387501 A | 14 May 2001 |
| | | AR 026310 A1 | 05 February 2003 |
| | | ES 2240197 T3 | 16 October 2005 |
| | | DE 19953185 A1 | 23 May 2001 |
| | | EP 1230148 A1 | 14 August 2002 |
| | | CA 2389428 A1 | 10 May 2001 |
| | | AT 292602 T | 15 April 2005 |
| | | BR 0015363 A | 18 June 2002 |
| | | TW 500692 B | 01 September 2002 |
| | | US 6447744 B1 | 10 September 2002 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107563051 A
- CN 109684769 A
- CN 107346378 A
- CN 107335390 A
- CN 107561938 A
- CN 106187660 A